# EUROPEAN PATENT APPLICATION

(11) **EP 3 871 620 A1**
(43) Date of publication of application: **01.09.2021**
(21) Application number: 21159081.5
(22) Date of filing: 24.02.2021
(51) Int. Cl.: A61B 17/34

(54) **SURGICAL ACCESS ASSEMBLY HAVING A BUTTON INFLATOR**

(30) Priority: 25.02.2020 US 202016800566
(71) Applicant: Covidien LP, Mansfield, MA 02048 (US)
(72) Inventor: DESJARDIN, Kevin, Prospect, CT 06712 (US); LOBO, Astley C., West Haven, CT 06516 (US); TOKARZ, Christopher A., Wallingford, CT 06492 (US); PATTISON, Douglas M., East Hartford, CT 06108 (US); BUYDA, Oksana, East Haven, CT 06513 (US); ADINOLFI, Amanda M., Wallingford, CT 06492 (US)
(74) Representative: Maschio, Antonio

(57) **Abstract**

A surgical access assembly includes elongated cannula member having proximal end portion and a distal end portion, a cannula housing coupled to the proximal end portion of the elongated cannula member, a balloon anchor coupled to the distal end portion of the elongated cannula member, and a collar having a port disposed along the elongated cannula member and in fluid communication with the balloon anchor. The port includes a first check valve, a second check valve, an actuatable button, and a release valve. The first check valve is disposed between a first end portion of the port and a second end portion of the port thereby defining a first chamber. The second check valve is coupled to the second end portion of the port and defines a second chamber between the second check valve and the first check valve. The actuatable button is configured to control flow of air into the balloon anchor. The release valve is configured to control flow of air out of the balloon anchor.

## Description

### FIELD

The present disclosure is generally related to surgical access devices and more particularly to a surgical access assembly having a button inflator for use in a minimally invasive surgical procedure.

### BACKGROUND

Minimally invasive surgical procedures including both endoscopic and laparoscopic procedures permit surgery to be performed on organs, tissues, and vessels far removed from an opening within the tissue. In laparoscopic procedures, the abdominal cavity is insufflated with an insufflation fluid, e.g., CO₂, to create a pneumoperitoneum thereby providing access to the underlying organs. A laparoscopic instrument is introduced through a cannula accessing the abdominal cavity to perform one or more surgical tasks. An interior of the cannula usually includes a seal to establish a substantially fluid-tight seal about the instrument to preserve the integrity of the pneumoperitoneum.

While minimally invasive surgical procedures have proven to be quite effective in surgery, several limitations remain. For example, the cannula which is subjected to the pressurized environment, i.e., the pneumoperitoneum, may exhibit a tendency to back out of the incision in the abdominal wall particularly during manipulation of the instrument within the cannula. Conventional cannulas may incorporate an inflatable balloon at the end of the cannula in an effort to resist withdrawal of the cannula from the tissue site. A pump may be coupled to the cannula and actuated to either inflate or deflate the balloon.

### SUMMARY

The present disclosure relates to a surgical access assembly including a balloon cannula for providing access to a surgical cavity within a patient (e.g., an abdominal cavity) having a button integrally coupled to the balloon cannula for inflating a balloon anchor of the balloon cannula.

In one aspect, the present disclosure provides a surgical access assembly including an elongated cannula member having proximal end portion and a distal end portion, a cannula housing coupled to the proximal end portion of the elongated cannula member, a balloon anchor coupled to the distal end portion of the elongated cannula member, and a collar having a port disposed along the elongated cannula member and in fluid communication with the balloon anchor. The port includes a first check valve, a second check valve, an actuatable button, and a release valve. The first check valve is disposed between a first end portion of the port and a second end portion of the port thereby defining a first chamber. The second check valve is coupled to the second end portion of the port and defines a second chamber between the second check valve and the first check valve. The actuatable button is configured to control flow of air into the balloon anchor. The release valve is configured to control flow of air out of the balloon anchor.

In aspects, the button may be in communication with the second chamber.

In aspects, the release valve may be in communication with the first chamber.

In aspects, one of the first or second check valves may be configured to transition between open and closed states in response to actuation of the button.

In aspects, transitioning the button from the first state to the second state may transition the first check valve to the open state and supply air to the balloon anchor.

In aspects, transitioning the button from the second state to the first state may transition the first check valve to the closed state.

In aspects, the closed state of the first check valve may maintain air pressure in the balloon anchor.

In aspects, transitioning the button to the first state may transition the second check valve to the open state and supply air to the second chamber and the button.

In aspects, the release valve may include a release check valve configured to control flow of air out of the balloon anchor and a piston in communication with the release check valve including a longitudinal tube therethrough.

In aspects, the release check valve may be configured to transition between open and closed states in response to actuation of the piston.

In aspects, the piston may be spring-loaded and configured to transition between an expanded state and a compressed state.

In aspects, the actuation of the piston may be configured to transition the release check valve to the open state, thereby releasing air from the balloon anchor.

In another aspect, the disclosure provides a method of inflating and deflating a balloon anchor of a surgical access assembly including actuating a button disposed on a collar of the surgical access assembly between a first check valve and a second check valve; supplying air to the balloon anchor; and maintaining air pressure in the balloon anchor.

In aspects, the method may include actuating a release valve coupled to the collar of the surgical access assembly and in communication with a second chamber; and releasing air from the balloon anchor.

In aspects, actuating the button may include transitioning the button between first and second states and the first and second check valve between an open state and a closed state.

In aspects, actuating the release valve may include transitioning the release valve between expanded and compressed states.

In aspects, supplying air to the balloon anchor may include transitioning the button from the first state to the second state and transitioning the first check valve from the closed state to the open state.

In aspects, maintaining air pressure in the balloon anchor may include transitioning the button from the second state to the first state and transitioning the first check valve to the closed state.

In aspects, releasing air from the balloon anchor may include transitioning the release valve from an expanded state to a compressed state and transitioning a release check valve of the release valve from a closed state to an open state.

In another aspect, the disclosure provides a surgical access assembly, including a balloon cannula having an elongated cannula member with proximal and distal end portions, and a cannula housing having an outer sleeve. The cannula housing is coupled to the proximal end portion of the elongated cannula member, and a balloon anchor is coupled to the distal end portion of the elongated cannula member formed with the outer sleeve. A collar is disposed along the elongated cannula member and in fluid communication with the balloon anchor, and includes a port extending outwardly from the elongated cannula member. The port includes a first end portion, a second end portion, a first check valve, a second check valve, an actuatable button, and a release valve. The first check valve is disposed between the first end portion of the port and the second end portion of the port. The first check valve and the port define a first chamber. The second check valve is coupled to the second end portion of the port. The second check valve and the port define a second chamber between the second check valve and the first check valve. The actuatable button is configured to control flow of air into the balloon anchor. The release valve is configured to control flow of air out of the balloon anchor.

The details of one or more aspects of the surgical access assemblies in accordance with the present disclosure are set forth in the accompanying drawings and the description below. Other features, objects, and advantages of the surgical access assemblies described in this disclosure will be apparent from the description and drawings, and from the claims.

### BRIEF DESCRIPTION OF DRAWINGS

FIG. 1 is a perspective view an exemplary surgical access assembly according to the present disclosure;
FIG. 2 is side cross-sectional view taken along section line 2-2 of FIG. 1;
FIG. 3 is an enlarged top cross-sectional view taken along section line 3-3 of FIG. 2;
FIG. 4 is an enlarged view of the detail indicated in FIG. 2;
FIG. 5 is a side cross-sectional view taken along section line 5-5 of FIG. 2;
FIG. 6 is an enlarged view of the area of detail indicated in FIG. 5;
FIG. 7 is an enlarged view of the area of detail indicated in FIG. 5;
FIG. 8 is the view of the area of detail indicated in FIG. 3 showing a button in a second state;
FIG. 9 is a side cross-sectional view taken along section line 9-9 of FIG. 8 showing the button in the second state;
FIG. 10 is the view of the area of detail indicated in FIG. 3, showing the button in the first state;
FIG. 11 is a side cross-sectional view taken along section line 11-11 of FIG. 10 showing the button in the second state;
FIG. 12 illustrates the inflated balloon anchor of FIG. 7;
FIG. 13 is a side view of the surgical access assembly of FIG. 1, wherein the surgical access assembly is inserted into a patient and the balloon anchor is inflated; and
FIG. 14 is an enlarged view of the area of detail indicated in FIG. 2 showing a release valve in a compressed state.

### DETAILED DESCRIPTION

Aspects of the presently disclosed surgical access assembly are described in detail with reference to the drawings, in which like reference numerals designate identical or corresponding elements in each of the several views. As used herein, the term "distal" refers to that portion of the surgical access assembly or component thereof, farther from the user, while the term "proximal" refers to that portion of the surgical access assembly, or component thereof, closer to the user.

As used herein, the term "clinician" refers to a doctor, nurse, surgeon, or other care provider and may include support personnel. In the following description, well-known functions, or construction are not described in detail to avoid obscuring the disclosure in unnecessary detail.

In general, the present disclosure provides a button for use with a balloon cannula. The button is integrally coupled to the balloon cannula. Upon actuation and release of the button, air may enter the balloon of the balloon cannula. Upon actuation of a release valve coupled to the balloon cannula, air may be released from the balloon of the balloon cannula. In this way, a clinician may no longer need to attach an extra component, such as, for example a pump or a syringe, to the balloon cannula to inflate or deflate the balloon of the balloon cannula.

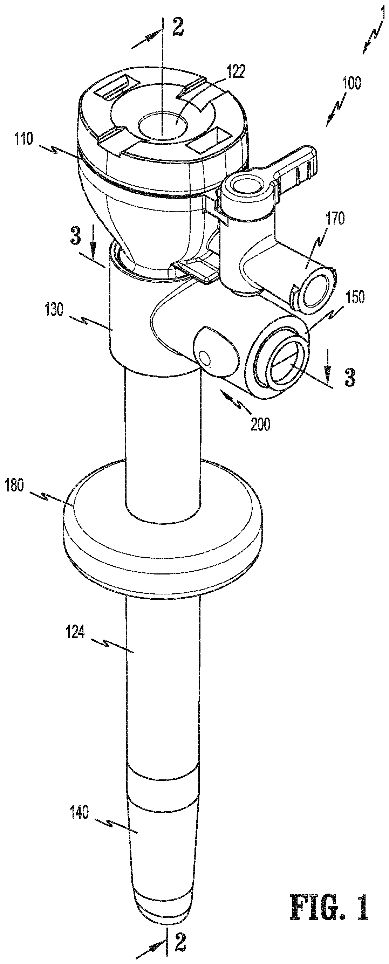

Referring initially to FIG. 1, which illustrates a surgical access assembly 1 generally including a surgical cannula assembly or balloon cannula 100, having an actuatable button or button 200 for use with the balloon cannula 100. The balloon cannula 100 is intended to permit access to an insufflated abdominal cavity during a minimally invasive (e.g., laparoscopic) procedure to permit the introduction of a surgical instrument for performing various surgical tasks on internal organs or structures within the cavity. The surgical instrument may be a surgical instrument such as laparoscopic or endoscopic clip appliers, graspers, dissectors, retractors, staplers, laser probes, photographic devices, tubes, endoscopes and laparoscopes, electro-surgical devices and the like. An obturator (not explicitly shown) may be positioned in the balloon cannula 100 to facilitate access to the abdominal cavity. The obturator may be any conventional obturator having a penetrating tip configured to penetrate tissue.

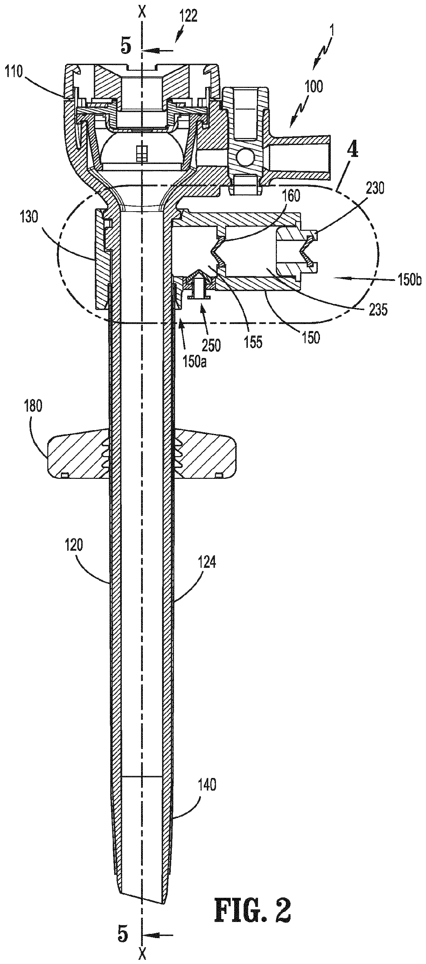

The balloon cannula 100 includes a cannula housing 110, an elongated cannula member 120 extending distally from the cannula housing 110, an outer sleeve 124 coaxially mounted over the elongated cannula member 120, a first collar 130, a second collar 180, and an expandable member or balloon anchor 140 formed with the outer sleeve 124. The cannula housing 110 is dimensioned for engagement by the clinician and may include or more internal seals (not shown) adapted to establish a seal about a surgical instrument introduced therethrough. The cannula housing 110 also may include an insufflation connector 170 (e.g., a luer connector) for connecting to a source of insufflation fluid (not shown) for delivery within, e.g., the abdominal cavity. The elongated cannula member 120 defines a longitudinal axis X-X (FIG. 2) along which the elongated cannula member 120 extends. The elongated cannula member 120 defines a longitudinal passageway 122 to permit passage of the surgical instrument. The longitudinal passageway 122 is also in fluid communication with the insufflation connector 170 to convey insufflation fluids into the abdominal cavity to establish and/or maintain the pneumoperi toneum.

With reference to FIG. 2, the first collar 130 of the balloon cannula 100 is positioned adjacent to the cannula housing 110 about the proximal end portion of the elongated cannula member 120. The first collar 130 has a tube or port 150 having a first end portion 150a and a second end portion 150b. The port 150 extends laterally outward from the first end portion 150a to the second end portion 150b and is configured to allow for the flow of air in and out of the balloon anchor 140. The port 150 also includes a first check valve 160, a second check valve 230, a release valve 250, and the button 200 (FIG. 1).

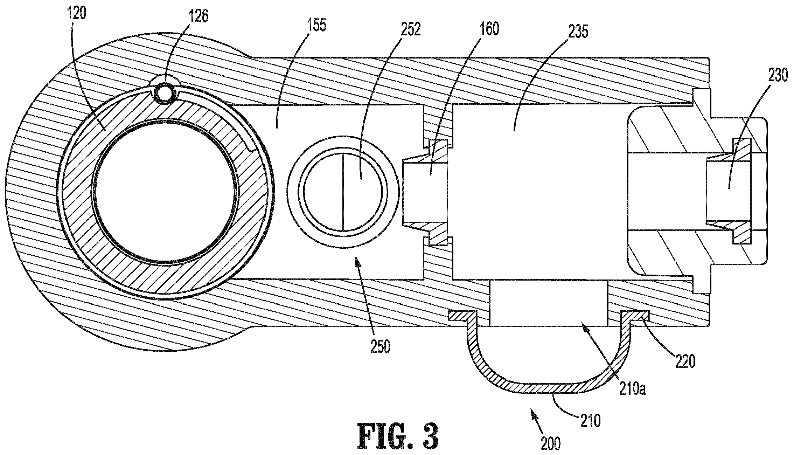

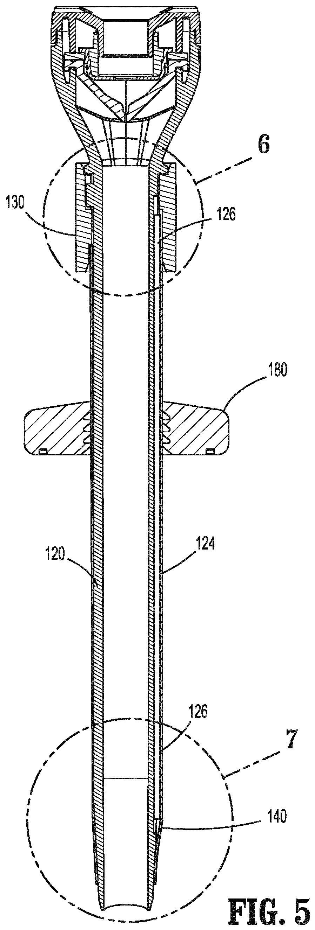

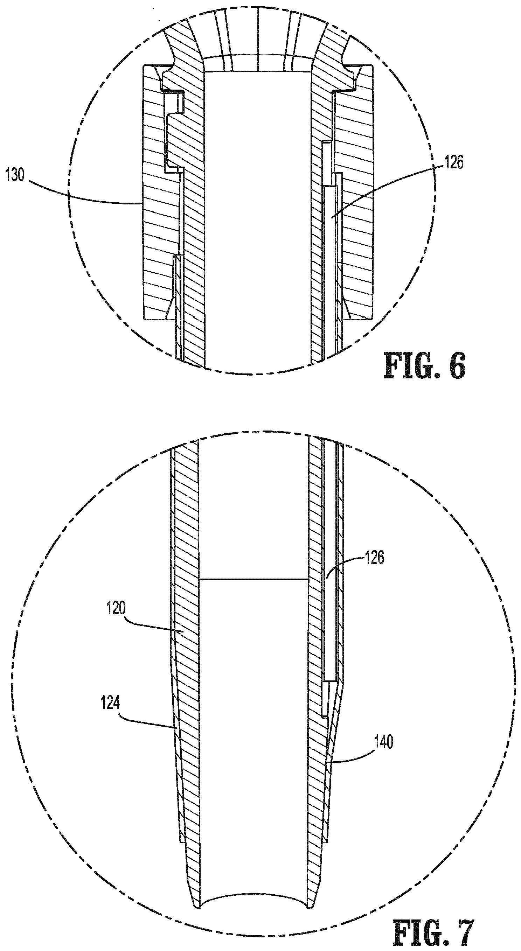

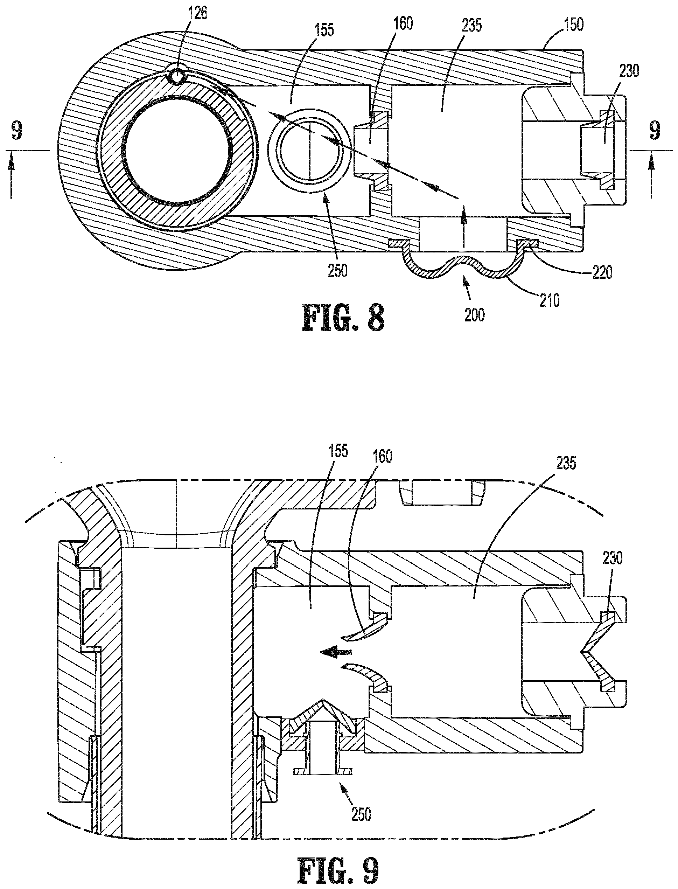

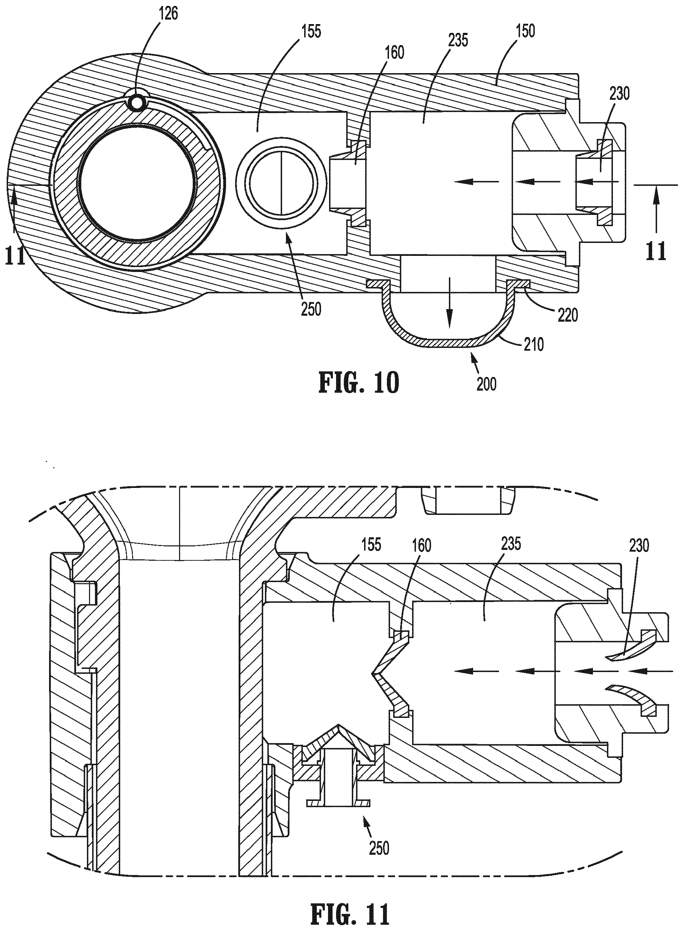

The port 150 is dimensioned to receive the first check valve 160 between the first end portion 150a and the second end portion 150b defining a first chamber 155 in the port 150 between the first check valve 160 and an outer surface of the elongated cannula member 120. The first chamber 155 is in fluid communication with the balloon anchor 140 via a lumen 126 (FIG. 3). The lumen 126, in some instances, may be a groove along the elongated cannula member 120. With quick reference to FIG. 5, the lumen 126, defined by the outer sleeve 124 and the elongated cannula member 120, extends from the first collar 130 (FIG. 6) distally towards the balloon anchor 140 (FIG. 7). The port 150 and the first check valve 160 may be ultrasonically welded together or mechanically engaged in some other fashion, e.g., snap-fit, adhesive, overmolded, etc. The first check valve 160 is configured to transition between open (FIG. 9) and closed (FIG. 11) states in response to mechanical actuation of the button 200, as will be described hereinbelow. The first check valve 160 may be any suitable type of valve, such as, for example, a diaphragm check valve, a swing check valve, a ball check valve, an in-line check valve, or a lift-check valve.

The port 150 is also dimensioned to receive the second check valve 230 at the second end portion 150b of the port 150 defining second chamber 235 in the port 150 between the second check valve 230 and the first check valve 160. The second check valve may be ultrasonically welded or mechanically engaged in some other fashion, e.g. snap-fit, adhesive, overmolded, etc. The second check valve 230 is configured to transition between open (FIG. 11) and closed (FIG. 9) states in response to mechanical actuation of the button 200, as will be described hereinbelow. The second check valve 230 may be any suitable type of valve, such as, for example, a diaphragm check valve, a swing check valve, a ball check valve, an in-line check valve, or a lift-check valve.

With reference to FIG. 3, the button 200 is configured to control the flow of air to the balloon anchor 140 and is in fluid communication with an opening 210a of the port 150 and the second chamber 235. The opening 210a is positioned between the first check valve 160 and the second check valve 230. The button 200 also includes a dome 210 and a flange 220. The flange 220 of the button 200 may be ultrasonically welded to the port 150 or mechanically engaged with one another in some other fashion, e.g., snap-fit, adhesive, overmolded, etc. The dome 210 of the button 200, when actuated, is configured to be transition between first (FIG. 10) and second (FIG. 8) states. The dome 210 of the button 200 may be soft and malleable and may be formed of any material with sufficient flex to allow for squeezing, such as, for example, rubber, silicone, vinyl, or neoprene. The dome 210 of the button 200 may have sufficient stiffness, based on material mechanical properties, to allow the dome 210 to return to an initial position and/or shape, thereby creating a vacuum inside the second chamber 235. It should be understood that the dome 210 of the button 200 may assume any geometry and/or shape suitable for depression and retention of air.

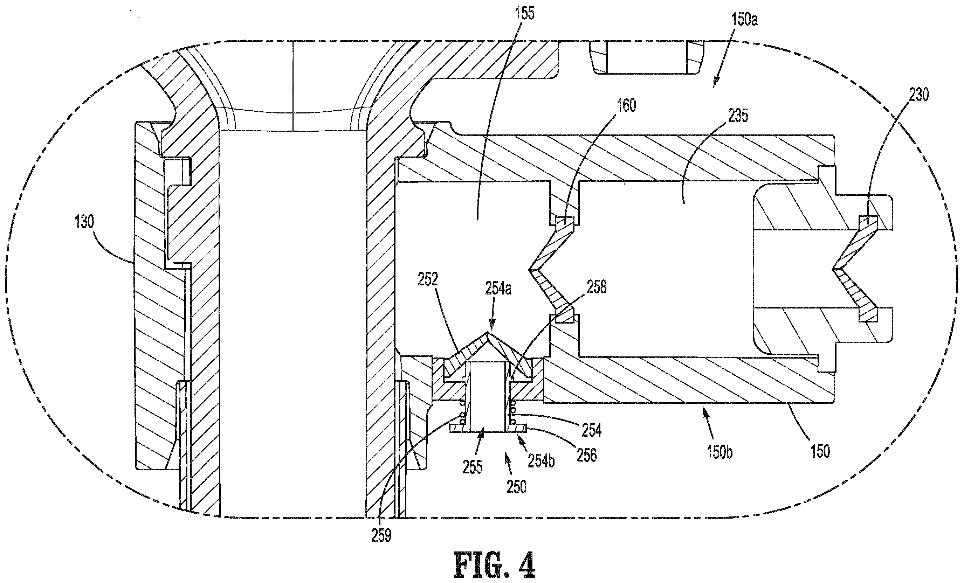

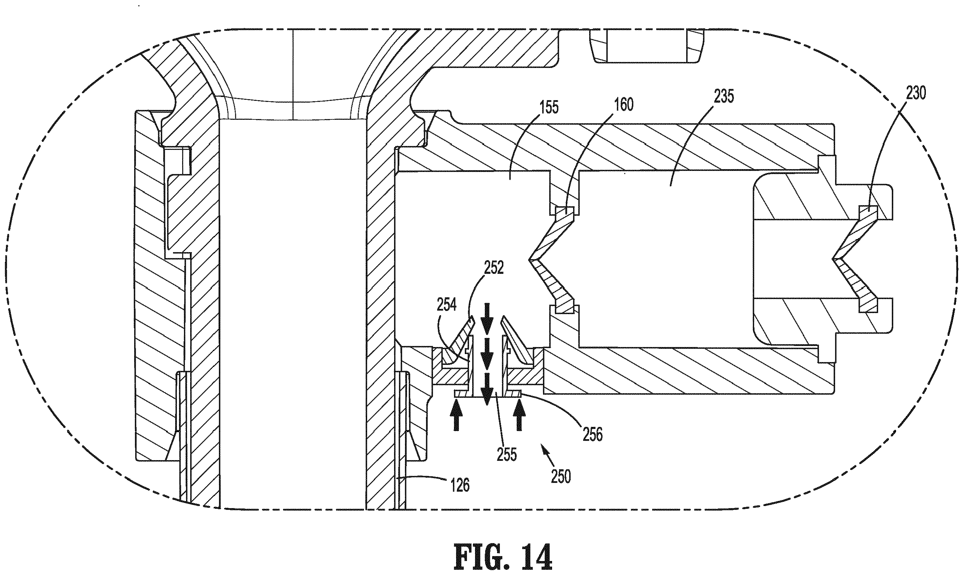

With reference to FIG. 4, the release valve 250 is disposed between the first end portion 150a of the port 150 and the second check valve 230 and is in fluid communication with the first chamber 155 to control the flow of air out of the balloon anchor 140. The release valve 250 includes a release check valve 252 and a piston 254. The release check valve 252 is configured to transition between open (FIG. 14) and closed (FIG. 4) states in response to actuation of the release valve 250. The release check valve 252 may be any suitable type of valve, such as, for example, a diaphragm check valve, a swing check valve, a ball check valve, an in-line check valve, or a lift-check valve. In some devices, the release valve 250 may be a quick release button configured to open the first check valve 160 and the second check valve 230, thus allowing air to be released from the balloon anchor 140 out the second check valve 230.

The piston 254 includes a longitudinal tube 255, a base 256, and a rib 258 disposed along an outer surface of the piston 254. The rib 258 circumscribes the outer surface of the piston 254, is disposed near a proximal end portion 254a of the piston 254, and is configured to prevent the piston 254 from being removed from the release valve 250. The base 256 has an opening that is fluidly coupled to a distal end portion 254b of the piston 254. The base 256 provides a surface for a clinician to depress and permit the flow of air out from the chamber 155 via the longitudinal tube 255 and the opening of the base 256 of the piston 254. The bottom end portion 150b of the port 150 is dimensioned to receive the piston 254, which includes a spring 259 that is spring-loaded and biased towards the expanded state (FIG. 4) and compressed state (FIG. 14) upon actuation of the piston 254 of the release valve 250.

In operation, the surgical access assembly 1 may be used in a minimally invasive surgery to provide access to an underlying cavity, e.g., an abdominal cavity. In one methodology, the abdominal cavity 30 is insufflated to establish a pneumoperitoneum. The obturator is positioned within the balloon cannula 100 and the assembled unit is advanced, while the balloon anchor 140 is in a deflated state, through a first layer of tissue 10 and a second layer of tissue 20, until the second collar 180 engages the first layer of tissue 10 (FIG. 11). In some instances, the assembled unit is advanced, while the balloon anchor 140 is in the deflated state, through the first layer of tissue 10, at which point the balloon anchor may be inflated to separate and/or dissect the first layer of tissue 10 and the second layer of tissue 20.

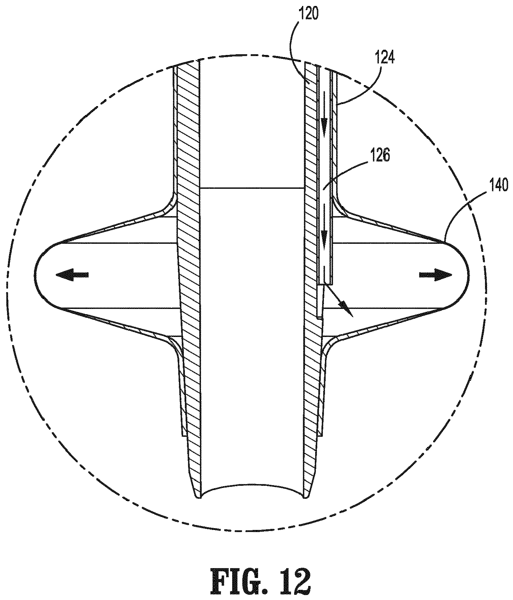

Referring to FIGS. 8-12, upon positioning the balloon anchor 140 adjacent the abdominal wall, the dome 210 of the button 200 is actuated, transitioning the button 200 from the first state (FIG. 3) to the second state (FIG. 8). Upon the button transitioning from the first state to the second state, the first check valve 160 is transitioned from the closed state (FIG. 4) to the open state (FIG. 9), thereby supplying air to the balloon anchor 140. The air is transferred from the button 200 to the first chamber 155 (FIG. 8) through the lumen 126 to the balloon anchor 140, causing the balloon anchor 140 to expand and inflate (FIG. 12). Once air is supplied to the balloon anchor 140, the button 200 is transitioned from the second state (FIG. 8) to the first state (FIG. 10) and the first check valve 160 is transitioned from the open state (FIG. 9) to the closed state (FIG. 11), thus maintaining air pressure in the balloon anchor 140 (FIG. 12). Air pressure is maintained in the balloon anchor 140, through the lumen 126 (FIG. 12) to the first chamber 155. In aspects, upon transitioning the button 200 from the second state to the first state, the second check valve 230 is transitioned from the closed state (FIG. 9) to the open state (FIG. 11), thus supplying air from the surrounding environment into the second chamber 235 and the button 200. The air from the surrounding environment fills the second chamber 235 and the button 200 with air, and the air pressure in the button 200 thereby causes the second check valve 230 to transition from the open state (FIG. 11) to the closed state (FIG. 9).

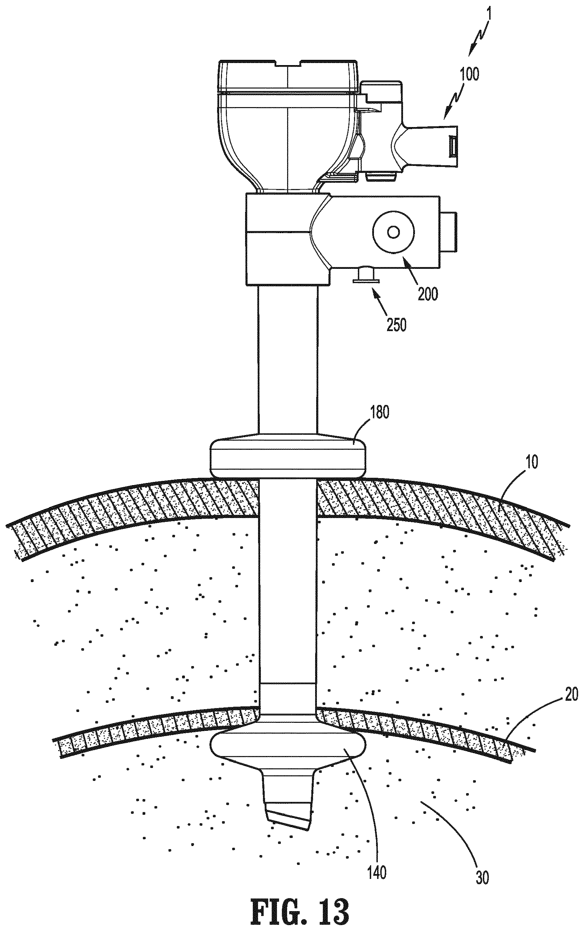

In some procedures, the button 200 may be rapidly transitioned between the first state and the second state, until the balloon anchor 140 is inflated to a desired size. In the inflated or at least partially inflated state, the balloon anchor 140 will resist withdrawal of the balloon cannula 100 from the abdominal cavity 30 while also providing a seal within the internal surface of the second layer of tissue 20, minimizing passage of fluids, including inflation fluids, from the abdominal cavity 30 (FIG. 13).

Referring to FIG. 14, to deflate and withdraw the balloon cannula 100 from the abdominal cavity 30 (FIG. 13), the release valve 250 is actuated transitioning the release valve 250 from the expanded state (FIG. 4) to the compressed state (FIG. 14). Once the release valve 250 is in the compressed state, the piston 254 engages the release check valve 252 transitioning the release check valve 252 from the closed state (FIG. 4) to the open state (FIG. 14). Upon, transitioning the release check valve 252 to the open state, air is released from the balloon anchor 140 through the lumen 126 (FIG. 5) into the chamber 155 and out the release valve 250 (FIG. 14). In some procedures, the release valve 250 may be transitioned to the compressed state and held until the balloon anchor 140 is fully deflated or at least partially deflated to allow for removal of the balloon cannula 100 from the abdominal cavity 30. In other procedures, the release valve 250 may be actuated and held, transitioning the first check valve 160 and the second check valve 230 from the closed position to the open position until the balloon anchor 140 is fully deflated or at least partially deflated to allow for removal of the balloon cannula 100. Once deflated, the balloon cannula 100 may be withdrawn from the abdominal cavity 30 through the second layer of tissue 20 and the first layer of tissue 10.

It should be understood that various features of the access assemblies specifically disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

The invention may be described by reference to the following numbered paragraphs:-
1. A surgical access assembly comprising:
   an elongated cannula member having proximal and distal end portions;
   a cannula housing coupled to the proximal end portion of the elongated cannula member;
   a balloon anchor coupled to the distal end portion of the elongated cannula member; and
   a collar having a port disposed along the elongated cannula member and in fluid communication with the balloon anchor, the port including:
      a first check valve disposed between a first end portion of the port and a second end portion of the port, wherein the first check valve and the port thereby defining a first chamber;
      a second check valve coupled to a second end portion of the port, wherein the second check valve and the port defines a second chamber between the second check valve and the first check valve;
      an actuatable button configured to control flow of air into the balloon anchor; and
      a release valve configured to control flow of air out of the balloon anchor.
2. The surgical access assembly of paragraph 1, wherein the button is in communication with the second chamber.
3. The surgical access assembly of paragraph 1, wherein the valve is in communication with the first chamber.
4. The surgical access assembly of paragraph 1, wherein one of the first or second check valves is configured to transition between open and closed states in response to actuation of the button.
5. The surgical access assembly of paragraph 4, wherein transitioning the button from the first state to the second state transitions the first check valve to the open state and supplies air to the balloon anchor.
6. The surgical access assembly of paragraph 5, wherein transitioning the button from the second state to the first state transitions the first check valve to the closed state.
7. The surgical access assembly of paragraph 6, wherein the closed state of the second check valve maintains air pressure in the balloon anchor.
8. The surgical access assembly of paragraph 7, wherein transitioning the button to the first state transitions the second check valve to the open state and supplies air to the second chamber and the button.
9. The surgical access assembly of paragraph 1, wherein the release valve includes a release check valve configured to control flow of air out of the balloon anchor and a piston in communication with the release check valve including a longitudinal tube therethrough.
10. The surgical access assembly of paragraph 8, wherein the release check valve is configured to transition between open and closed states in response to actuation of the piston.
11. The surgical access assembly of paragraph 8, wherein the piston is spring-loaded and configured to transition between an expanded state and a compressed state.
12. The surgical access assembly of paragraph 9, wherein the actuation of the piston is configured to transition the release check valve to the open state, thereby releasing air from the balloon anchor.
13. A method of inflating and deflating a balloon anchor of a surgical access assembly comprising:
   actuating a button disposed on a collar of the surgical access assembly between a first check valve and a second check valve;
   supplying air to the balloon anchor; and
   maintaining air pressure in the balloon anchor.
14. The method of paragraph 13, further comprising:
   actuating a release valve coupled to the collar of the surgical access assembly and in communication with a first chamber; and
   releasing air from the balloon anchor.
15. The method of paragraph 13, wherein actuating the button includes transitioning the button between first and second states and the first and second check valve between an open state and a closed state.
16. The method of paragraph 14, wherein actuating the release valve includes transitioning the release valve between expanded and compressed states.
17. The method of paragraph 13, wherein supplying air to the balloon anchor includes transitioning the button from the first state to the second state and transitioning the first check valve from the closed state to the open state.
18. The method of paragraph 13, wherein maintaining air pressure in the balloon anchor includes transitioning the button from the second state to the first state and transitioning the first check valve to the closed state.
19. The method of paragraph 14, wherein releasing air from the balloon anchor includes transitioning the release valve from an expanded state to a compressed state and transitioning a release check valve of the release valve from a closed state to an open state.
20. A surgical access assembly, comprising:
   a balloon cannula including:
      an elongated cannula member having proximal and distal end portions;
      a cannula housing having an outer sleeve, the cannula housing coupled to the proximal end portion of the elongated cannula member;
      a balloon anchor formed with the outer sleeve and coupled to the distal end portion of the elongated cannula member; and
      a collar disposed along the elongated cannula member and in fluid communication with the balloon anchor, the collar including:
         a port extending outwardly from the elongated cannula member, the port configured having a first end portion and a second end portion;
         a first check valve disposed between the first end portion of the port and the second end portion of the port, wherein the first check valve and the port define a first chamber;
         a second check valve coupled to the second end portion of the port, wherein the second check valve and the port define a second chamber between the second check valve and the first check valve;
         an actuatable button configured to control flow of air into the balloon anchor; and
         a release valve configured to control flow of air out of the balloon anchor.

## Claims

1. A surgical access assembly comprising:
an elongated cannula member having proximal and distal end portions;
a cannula housing coupled to the proximal end portion of the elongated cannula member;
a balloon anchor coupled to the distal end portion of the elongated cannula member; and
a collar having a port disposed along the elongated cannula member and in fluid communication with the balloon anchor, the port including:
a first check valve disposed between a first end portion of the port and a second end portion of the port, wherein the first check valve and the port thereby defining a first chamber;
a second check valve coupled to a second end portion of the port, wherein the second check valve and the port defines a second chamber between the second check valve and the first check valve;
an actuatable button configured to control flow of air into the balloon anchor; and
a release valve configured to control flow of air out of the balloon anchor.

2. The surgical access assembly of claim 1, wherein the button is in communication with the second chamber.

3. The surgical access assembly of claim 1 or claim 2, wherein the valve is in communication with the first chamber.

4. The surgical access assembly of any preceding claim, wherein one of the first or second check valves is configured to transition between open and closed states in response to actuation of the button; preferably wherein transitioning the button from the first state to the second state transitions the first check valve to the open state and supplies air to the balloon anchor.

5. The surgical access assembly of claim 4, wherein transitioning the button from the second state to the first state transitions the first check valve to the closed state; preferably wherein the closed state of the second check valve maintains air pressure in the balloon anchor.

6. The surgical access assembly of claim 7, wherein transitioning the button to the first state transitions the second check valve to the open state and supplies air to the second chamber and the button.

7. The surgical access assembly of any preceding claim, wherein the release valve includes a release check valve configured to control flow of air out of the balloon anchor and a piston in communication with the release check valve including a longitudinal tube therethrough; preferably wherein the release check valve is configured to transition between open and closed states in response to actuation of the piston.

8. The surgical access assembly of claim 7, wherein the piston is spring-loaded and configured to transition between an expanded state and a compressed state; preferably wherein the actuation of the piston is configured to transition the release check valve to the open state, thereby releasing air from the balloon anchor.

9. A method of inflating and deflating a balloon anchor of a surgical access assembly comprising:
actuating a button disposed on a collar of the surgical access assembly between a first check valve and a second check valve;
supplying air to the balloon anchor; and
maintaining air pressure in the balloon anchor.

10. The method of claim 9, further comprising:
actuating a release valve coupled to the collar of the surgical access assembly and in communication with a first chamber; and
releasing air from the balloon anchor.

11. The method of claim 9 or claim 10, wherein actuating the button includes transitioning the button between first and second states and the first and second check valve between an open state and a closed state.

12. The method of any of claims 10 or 11, wherein actuating the release valve includes transitioning the release valve between expanded and compressed states.

13. The method of any of claims 9 to 12, wherein supplying air to the balloon anchor includes transitioning the button from the first state to the second state and transitioning the first check valve from the closed state to the open state.

14. The method of any of claims 9 to 13, wherein maintaining air pressure in the balloon anchor includes transitioning the button from the second state to the first state and transitioning the first check valve to the closed state; and/or wherein releasing air from the balloon anchor includes transitioning the release valve from an expanded state to a compressed state and transitioning a release check valve of the release valve from a closed state to an open state.

15. A surgical access assembly, comprising:
a balloon cannula including:
an elongated cannula member having proximal and distal end portions;
a cannula housing having an outer sleeve, the cannula housing coupled to the proximal end portion of the elongated cannula member;
a balloon anchor formed with the outer sleeve and coupled to the distal end portion of the elongated cannula member; and
a collar disposed along the elongated cannula member and in fluid communication with the balloon anchor, the collar including:
a port extending outwardly from the elongated cannula member, the port configured having a first end portion and a second end portion;
a first check valve disposed between the first end portion of the port and the second end portion of the port, wherein the first check valve and the port define a first chamber;
a second check valve coupled to the second end portion of the port, wherein the second check valve and the port define a second chamber between the second check valve and the first check valve;
an actuatable button configured to control flow of air into the balloon anchor; and
a release valve configured to control flow of air out of the balloon anchor.
